# EUROPEAN PATENT APPLICATION

(11) **EP 4 218 713 A1**
(43) Date of publication of application: **02.08.2023**
(21) Application number: 22153581.8
(22) Date of filing: 27.01.2022
(51) Int. Cl.: A61K 8/31, A61K 8/34, A61K 8/36, A61Q 19/00

(54) **USE OF A TOPICAL SKIN CARE COMPOSITION FOR ENHANCING STRATUM CORNEUM LIPID BIOSYNTHESIS**

(71) Applicant: Unilever IP Holdings B.V., 3013 AL Rotterdam (NL); Unilever Global IP Limited, Wirral, Merseyside CH62 4ZD (GB)
(72) Inventor: The designation of the inventor has not yet been filed
(74) Representative: James, Helen Sarah

(57) **Abstract**

The invention provides the cosmetic use of a topically applied skin care composition comprising glycerol, C₁₆₋₁₈ fatty acid and petrolatum for enhancing stratum corneum (SC) lipid biosynthesis by resident skin commensal microbes.

## Description

### Field of the Invention

The present invention relates to the use of a topical skin care composition for enhancing stratum corneum lipid biosynthesis.

### Background of the Invention

The stratum corneum contains layers of corneocytes embedded in a lipid matrix which is made up of a unique complex mixture of polar and nonpolar lipids that, unlike biological membranes, is almost devoid of phospholipids. Its main components are ceramides, cholesterol, and free fatty acids (predominantly long-chain and saturated) which are organized into specific lamellar structures whose integrity depends not only on the quality of the fractions present but also on their respective proportions.

Lipids in the stratum corneum are considered to play a crucial role in healthy skin form and function. Lower levels of lipids, especially longer chain (C₂₀-C₂₆) free fatty acids have been reported in compromised skin and are thought to result in a "leaky" barrier with an increase of trans-epidermal water loss (TEWL). In xerosis (commonly referred to as dry skin), a strong correlation between dry skin attributes (conductance, dryness, roughness and scaliness) and the level of ceramides has also been observed.

Evidently it is important to be able to stimulate the synthesis of stratum corneum lipids so as to maintain and/or restore their integrity, thus allowing them to carry out the important roles for which they are responsible.

The present invention addresses this problem.

### Summary of the Invention

The invention provides the cosmetic use of a topically applied skin care composition comprising glycerol, C₁₆₋₁₈ fatty acid and petrolatum for enhancing stratum corneum (SC) lipid biosynthesis by resident skin commensal microbes.

### Detailed Description of the Invention

As used herein, "skin care" means regulating and/or improving cosmetic qualities of the skin, (as opposed to curing, treating, or preventing a disease or disorder). Accordingly, such cosmetic qualities are subject to regulation and/or improvement both in healthy subjects as well as those which present diseases or disorders of the skin (such as psoriasis, lichen planus, folliculitis, or atopic dermatitis).

Examples of "skin care" benefits in the context of this invention include providing a smoother, more even texture; improving the elasticity or resiliency of the skin; improving the firmness of the skin; improving the hydration status or moisturization of the skin; improving skin barrier properties; and reducing the appearance of redness or skin blotches.

The topically applied skin care composition for use in the invention comprises glycerol, C₁₆₋₁₈ fatty acid and petrolatum, typically in combination with a cosmetically acceptable vehicle.

Glycerol (also referred to as glycerine, glycerin or 1,2,3-propanetriol) acts to increase and maintain moisture in the skin by attracting water to the stratum corneum of the epidermis. The amount of glycerol suitably ranges from about 1 to about 25%, preferably from about 2 to about 15% (by weight based on the total weight of the composition).

As used herein, the term " C₁₆₋₁₈ fatty acid" refers to a carboxylic acid having a linear or branched aliphatic tail of 16 to 18 carbon atoms. The aliphatic tail can be saturated, monounsaturated, or polyunsaturated. Examples of fatty acids for use in the topically applied skin care composition for use in the invention include palmitic acid (C16), palmitoleic acid (C16:1), stearic acid (C18), isostearic acid (C18), oleic acid (C18:1), linoleic acid (C18:2), *α-*linolenic (C18:3, *ω*-3) and *γ*-linolenic (C18:3, *ω*-6). Preferred examples of C₁₆₋₁₈ fatty acids for use in the topically applied skin care composition for use in the invention are stearic acid and palmitic acid.

Mixtures of any of the above-described materials may also be used.

The amount of C₁₆₋₁₈ fatty acid suitably ranges from about 0.5 to about 6%, preferably from about 1.5 to about 4% (by weight based on the total weight of the composition).

Petrolatum (also known as mineral wax, petroleum jelly or mineral jelly) acts to reduce trans-epidermal water loss (TEWL). Petrolatum is a purified mixture of semisolid saturated hydrocarbons having the general formula CₙH₂ₙ₊₂ and is obtained from petroleum. The hydrocarbons consist mainly of branched and unbranched chains although some cyclic alkanes and aromatic molecules with paraffin side chains may also be present. Various grades of petrolatum are commercially available, which vary in their physical properties depending upon their source and refining process. A preferred grade of petrolatum for use in the invention is white petrolatum.

Mixtures of any of the above-described materials may also be used.

The amount of petrolatum suitably ranges from about 0.1 to about 10%, preferably from about 0.2 to about 5% (by weight based on the total weight of the composition).

The term "cosmetically acceptable vehicle" means that the vehicle is suitable for topical application to the skin, has good aesthetic properties and will not cause any safety or toxicity concerns.

The cosmetically acceptable vehicle may comprise an aqueous phase, an oil phase, an alcohol, a silicone phase, or a mixture thereof, and may be in the form of an emulsion. Emulsions can have a range of consistencies including thin lotions (which may also be suitable for spray or aerosol delivery) and creamy lotions.

Combinations of any of the above-described product forms may also be used.

Exemplary emulsions suitable for use in the invention include water-in-oil emulsions, oil-in-water emulsions, silicone-in-water emulsions, water-in-silicone emulsions, polyol-in-silicone emulsions, silicone-in-polyol emulsions, polyol-in-oil emulsions, oil-in-polyol emulsions, wax-in-water emulsions, and water-oil-water triple emulsions. Preferred emulsions include oil-in-water emulsions and water-in-oil emulsions, more preferably oil-in-water emulsions.

Exemplary emulsions suitable for use in the invention typically have an oil phase containing at one or more cosmetically acceptable fatty materials which may be liquid or solid at room temperature (25°C).

Suitable cosmetically acceptable fatty materials include naturally derived oils (such as sunflower oil, borage oil, soybean oil, castor oil, olive oil and almond oil); hydrogenated naturally derived oils which are solid at 25° C (such as hydrogenated castor oil, hydrogenated jojoba oil, hydrogenated palm oil, hydrogenated tallow and hydrogenated coconut oil) ; esters of monoalcohols or of glycols with monocarboxylic or polycarboxylic acids, at least one of the alcohols and/or acids comprising at least one hydrocarbon-based chain containing at least 6 carbon atoms (such as octyl palmitate, isopropyl myristate, isopropyl palmitate, isopropyl isostearate, hexyl laurate, isohexyl laurate, isohexyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, isononyl isononanoate, propylene glycol dicaprate, diisopropyl adipate, dibutyl adipate, and oleyl adipate); ethers (such as dicapryl ether); fatty alcohols (such as cetyl alcohol, stearyl alcohol and behenyl alcohol); isoparaffins (such as isooctane, isododecane and isohexadecane); silicone oils (such as dimethicones, cyclic silicones, and polysiloxanes); and vegetable fats (such as cocoa butter, coconut oil, palm oil and shea butter).

Mixtures of any of the above-described materials may also be used.

Topical cosmetic compositions in the form of an emulsion, and suitable for use in the invention, typically have an aqueous phase, with the amount of water in such an emulsion suitably ranging from about 5 to about 95%, preferably from about 35 to about 80% (by weight based on the total weight of the composition). The aqueous phase may also include one or more organic liquids that are miscible with water at room temperature (25°C). Exemplary water-miscible organic liquids include monohydric and polyhydric alcohols and derivatives thereof such as C₂-C₆ alkanols (such as ethanol and isopropanol); C₂-C₁₀ glycols (such as propylene glycol, butylene glycol, pentylene glycol, hexylene glycol, caprylyl glycol, dipropylene glycol, and diethylene glycol); C₃-C₁₆ glycol ethers (such as mono-, di-, or tripropylene glycol (C₁-C₄) alkyl ethers and mono-, di-, or triethylene glycol (C₁-C₄) alkyl ethers) and polyethylene glycol having 2 to 12 oxyethylene units.

Topical cosmetic compositions in the form of an emulsion, and suitable for use in the invention, generally include emulsifiers and solubilizers, to enable two or more immiscible components to be combined homogeneously and to help stabilize the composition. The amount of emulsifier and solubilizer in such an emulsion suitably ranges from about 0.1 to about 30%, preferably from about 1 to about 8% (by weight based on the total weight of the composition). Emulsifiers that may be used to form O/W or W/O emulsions include sorbitan oleate, sorbitan sesquioleate, sorbitan isostearate, sorbitan trioleate, PEG-20 sorbitan isostearate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, polyglyceryl-2 dipolyhydroxystearate, PEG-30 dipolyhydroxystearate, oleamide DEA, TEA myristate, TEA stearate, magnesium stearate, sodium stearate, potassium laurate, potassium ricinoleate, sodium cocoate, sodium tallowate, potassium castorate, sodium oleate, cetyl phosphate, diethanolamine cetyl phosphate, potassium cetyl phosphate, sodium glyceryl oleate phosphate, dimethicone copolyol, cetyl dimethicone copolyol, octyldimethicone ethoxyglucoside copolyol, dimethicone copolyol crosspolymer and laurylmethicone copolyol.

Mixtures of any of the above-described materials may also be used.

### Skin Care Actives

A topical cosmetic composition for use in the invention may include additional skin care actives (for improving the physical and/or aesthetic characteristics of the skin). Examples of additional skin care actives which are suitable for use in the invention include vitamins, minerals and/or antioxidants, skin anti-hyperpigmentation agents, sunscreens, anti-irritants, exfoliating agents, and mixtures thereof.

Vitamins, minerals and/or antioxidants suitable for use in the invention include natural botanical antioxidants derived from plant materials such as fruits, vegetables, herbs and spices (such as goji berry, white tea, rosemary, green tea, grape seed and lemongrass extracts); vitamin A and its precursors or derivatives (such as beta-carotene, retinyl palmitate); vitamin B3 and its precursors or derivatives (such as niacinamide); vitamin B5 and its precursors or derivatives (such as panthenol and its precursors or derivatives); vitamin C and its precursors or derivatives (such as tetrahexyldecyl ascorbate, ascorbyl palmitate); vitamin E and its precursors or derivatives (such as d-alpha-tocopherol, tocopheryl acetate); vitamin K and its precursors or derivatives; selenium and its derivatives (such as L-selenomethionine); and alpha lipoic acid.

Skin anti-hyperpigmentation agents suitable for use in the invention include natural botanical agents derived from plant materials (such as *Arctostaphylos patula* and *Arctostaphylos viscida* extracts, *Emblica officinalis* extract, *Mitracarpus scaber* extract, *Uva ursi* (bearberry) extract, *Morus bombycis* (mulberry) extract, *Morus alba* (white mulberry) extract, *Broussonetia papyrifera* (paper mulberry) extract, licorice extract, acerola cherry extract, *Chlorella vulgaris* extract, *Aloe ferrox* extract and *Rumex occidentalis* extract); synthetic or natural sugar amines (such as glucosamine, N-acetyl glucosamine, glucosamine sulfate, mannosamine, N-acetyl mannosamine, galactosamine, N-acetyl galactosamine and their hydrochloride salts); retinoids (such as retinol, retinal, retinoic acid and C₂₋₂₀ esters of retinol such as retinyl palmitate, retinyl acetate, retinyl propionate, retinyl linoleate and retinyl oleate); kojic acid and C₂₋₂₀ esters thereof (such as kojic acid monobutyrate, kojic acid monocaprate, kojic acid monopalmitate, kojic acid monostearate and kojic acid monobenzoate); hydroquinone, hydroquinone monomethyl ether, hydroquinone monoethyl ether, hydroquinone monobenzyl ether, α and β-arbutin, deoxyarbutin (4-[(tetrahydro-2H-pyran-2-yl)oxy]phenol), mequinol (4-hydroxyanisole), glutathione, cysteine, N-acetyl-L-cysteine, azelaic acid, magnesium ascorbyl phosphate, sodium ascorbyl phosphate, peroxides (such as hydrogen peroxide, zinc peroxide, sodium peroxide and benzoyl peroxide); 3-aminotyrosine, glycyrrhizinic acid and 4-substituted resorcinol derivatives (such as 4-methyl resorcinol, 4-ethyl resorcinol, 4-propyl resorcinol, 4-isopropyl resorcinol, 4-butyl resorcinol, 4-pentyl resorcinol, 4-hexyl resorcinol, 4-heptyl resorcinol, 4-octyl resorcinol, 4-nonyl resorcinol, 4-decyl resorcinol, 4-undecyl resorcinol, 4-dodecyl resorcinol, 4-cyclopentyl resorcinol, 4-cyclohexyl resorcinol, 4-cycloheptyl resorcinol, and 4-cyclooctyl resorcinol).

Sunscreens suitable for use in the invention protect the skin from ultraviolet (UV) solar radiation falling within both the UVB region (between 290nm to 320 nm wavelengths) and the UVA region (between 320nm and 400nm wavelengths). Examples of sunscreens include methoxycinnamate derivatives (such as octyl methoxycinnamate and isoamyl methoxycinnamate); camphor derivatives (such as 4-methyl benzylidene camphor, camphor benzalkonium methosulfate, and terephthalylidene dicamphor sulfonic acid); salicylate derivatives (such as octyl salicylate and homosalate); sulfonic acid derivatives (such as phenylbenzimidazole sulfonic acid); benzone derivatives (such as dioxybenzone, sulisobenzone, and oxybenzone); benzoic acid derivatives (such as aminobenzoic acid and octyldimethyl para-amino benzoic acid); octocrylene, diethylhexyl butamido triazone, octyl triazone, butyl methoxydibenzoyl methane, drometrizole trisiloxane, menthyl anthranilate and inorganic UV-absorbing particles (such as zinc oxide and titanium dioxide).

Anti-irritants suitable for use in the invention include allantoin, aloe vera, α-bisabolol, caffeine, chamomile extract, Cola *nitada* extract, cucumber extract, dipotassium glycyrrhizinate, glycyrrhizic acid, green tea extract, lecithin or hydrogenated lecithin, licorice extract, *Avena sativa* (oat) meal extract, tea tree oil, salicylic acid, acetylsalicylic acid, strontium acetate, strontium chloride, strontium nitrate, fatty acids with anti-irritant properties (such as linoleic acid and linolenic acid) and aromatic aldehydes with anti-irritant properties (such as 4-methoxy benzaldehyde, 4-ethoxy benzaldehyde, 4-butoxy benzaldehyde and 4-pentoxy benzaldehyde).

Exfoliating agents suitable for use in the invention include benzoyl peroxide, benzoic acid, 3-hydroxy benzoic acid, salicylic acid, acetic acid, trichloroacetic acid, 1-pyrrolidone-5-carboxylic acid, α-hydroxy acids (such as glycolic acid, lactic acid, malic acid, tartaric acid, and citric acid); β-hydroxy acids (such as β-hydroxybutyric acid); α-keto acids (such as pyruvic acid, 2-oxopropanoic acid, 2-oxobutanoic acid and 2-oxopentanoic acid); and oxa acids (such as 3,6,9-trioxaundecanedioic acid).

Mixtures of any of the above-described materials may also be used.

### Functional Ingredients

A topical cosmetic composition for use in the invention may include additional functional ingredients (for improving the physical and/or aesthetic characteristics of the composition).

Examples of additional functional ingredients which are suitable for use in the invention include water soluble or colloidally water soluble polymeric thickening agents (such as hydroxyethyl cellulose, methyl cellulose, hydroxypropyl methyl cellulose, polyquaternium-10, carrageenan, guar gum, hydroxypropyl guar gum, xanthan gum, polyvinylalcohol, acrylic acid/ethyl acrylate copolymers, carboxyvinyl polymers, cross-linked polyacrylate polymers and polyacrylamide polymers); structurant clays (such as magnesium aluminum silicate, attapulgite, bentonite, montmorillonite and hectorite); inorganic pigments (such as titanium oxide, zirconium oxide, cerium oxide zinc oxide, iron oxide, chromium oxide and ferric blue); organic pigments (such as carbon black and barium, strontium, calcium, and aluminum lakes); pearlescent agents (such as mica coated with titanium oxide and/or iron oxide); dyes, preservatives (such as disodium EDTA, benzyl alcohol, methylparaben, phenoxyethanol, propylparaben, ethylparaben, butylparaben and isobutylparaben); pH adjusters and fragrances.

Mixtures of any of the above-described materials may also be used.

When making the topical cosmetic composition as described above, typically the constituent ingredients are mixed with moderate shear, under atmospheric conditions and at a temperature from 20 to 65°C.

The topical cosmetic composition as described above will typically have a viscosity under 25,000 cps, such as from 250 to 22,000, preferably from 350 to 13,000, more preferably from 500 to 10,000 cps. Viscosity may be measured with instrumentation such as a Brookfield Viscometer RVT, Model D220, using a T-bar spindle D at 5 RPM, 60 seconds at 25°C.

### Use

The topical cosmetic composition as described above is used for enhancing stratum corneum (SC) lipid biosynthesis by resident skin commensal microbes.

As used herein, the term "resident skin commensal microbes" denotes the diverse community of microbes which can normally be found on healthy human skin, whose interactions on the skin are either neutral or beneficial, and which are permanent inhabitants on the surface of the skin, the stratum corneum and within the outer layer of the epidermis and the deeper crevices of the skin and hair follicles. Microbial colonization is differentially shaped by the physiological and topological variation of the skin, and varies systematically among different skin habitats, such as between dry, moist, and sebaceous skin. Resident skin commensal microbes are characterized in that they can grow and multiply on the skin without invading or damaging the skin tissue. Another characteristic of these microbes is that washing does not easily remove them in deeper skin regions. These microbes, their combined genetic material, and the environment in which they live collectively form the human skin microbiome.

The human skin microbiome includes bacteria, fungi, yeasts, viruses, archaea, and mites. Bacteria are the most dominant group. Over 90% of bacteria of the human skin microbiome belong to four main phyla: *Actinobacteria, Firmicutes, Proteobacteria* and *Bacteroidetes.* The major identified genera are *Corynebacteria, Cutibacteria* and *Staphylococci,* whose representatives may constitute 45 to 80% of the entire skin microbiome, according to estimates.

As used herein, the term "stratum corneum (SC) lipids" denotes cholesterol, ceramides, and free fatty acids (FFAs), especially longer chain FFAs having a linear or branched, saturated or monounsaturated aliphatic tail of at least 20 carbon atoms, such as from 20 to 32 carbon atoms. Examples of such FFAs include arachidic acid (C20:0), behenic acid (C22:0), erucic acid (C22:1) and lignoceric acid (C24:0).

### Packaging

A topical cosmetic composition for use in the invention (as described above) may be packaged in a suitable container to suit its viscosity and intended use by the consumer. For example, a lotion or a cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar.

The composition is suitably applied to the skin at the rate of one or two applications per day. Generally, an amount corresponding to about 1 to 2 ml of the composition per application is applied uniformly over the area of treatment twice daily for a period of at least 7 (seven) days, more preferably at least 30 (thirty) days.

The invention will be further illustrated by the following, non-limiting Example, in which all percentages are by weight based on total weight unless otherwise specified.

### Examples

A study was carried out to examine the impact of 5 weeks use of a body lotion, comprising glycerol, C₁₆₋₁₈ fatty acid and petrolatum, on stratum corneum (SC) lipids and the skin microbiome. The body lotion had ingredients as shown in Table 1:

**Table 1**

| **Ingredient** | **wt.% (a.i.)** |
|---|---|
| Alkyl acrylate crosspolymer | 0.2 |
| Xanthan gum | 0.05 |
| Glycerin | 10 |
| Magnesium aluminum silicate | 0.3 |
| Stearic acid | 3.6 |
| Glycol stearate/stearamide AMP | 0.9 |
| Glyceryl monostearate | 0.8 |
| Cetyl alcohol | 0.5 |
| PEG stearate | 0.7 |
| Isopropyl myristate | 1.5 |
| Mineral oil | 1 |
| Dimethicone | 0.8 |
| Petrolatum | 0.5 |
| Tapioca starch | 0.5 |
| Hydroxyethyl urea | 0.02 |
| Water, fragrance, preservative, pH adjuster, chelating agent | to 100 |

Female subjects aged 18 to 55 years with moderate levels of skin dryness on both lower outer legs were recruited to the study. Baseline measurements of skin dryness were conducted, followed by sampling of the skin microbiome and the collection of tape strips. The subjects were then asked to apply the body lotion twice daily to one lower leg for 5 weeks after which subjects were resampled.

Free fatty acid (FFA) levels were measured in the outer-most 5 tape strips at both timepoints. A mixed effects model was used to assess the differences between timepoints. Total FFA (with standard deviation) increased from 14.7ng/ug protein (6.93ng/ug) to 36.94ng/ug protein (18.33ng/ug), p-value <0.001. Individual species analysis revealed significant increases in the levels of several FFAs from chain length C14:0 to C20:0 following the lotion application phase. Levels of C22:0 also increased although the increase was not statistically significant.

The results are shown in Table 2:

**Table 2**

| **Fatty acid** | **Baseline average** | **Standard deviation** | **Week 5 average** | **Standard deviation** | **p-value** |
|---|---|---|---|---|---|
| **C14:0** | 0.6 | 0.58 | 1.14 | 0.7 | <0.001 |
| **C16:0** | 3.82 | 2.01 | 14.55 | 8.83 | <0.001 |
| **C16:1** | 0.44 | 0.46 | 0.45 | 0.31 | 0.316 |
| **C18:0** | 2.99 | 1.26 | 13.42 | 7.52 | <0.001 |
| **C18:1** | 2.39 | 2.34 | 2.6 | 2.43 | 0.184 |
| **C18:2** | 1.02 | 0.58 | 1.32 | 0.65 | 0.002 |
| **C20:0** | 0.21 | 0.05 | 0.46 | 0.2 | <0.001 |
| **C22:0** | 0.44 | 0.1 | 0.47 | 0.1 | 0.086 |
| **C22:1** | 0 | 0 | 0.01 | 0.01 | 0.44 |
| **C24:0** | 1.63 | 0.41 | 1.58 | 0.46 | 0.902 |

Average levels for each ceramide class in pmol/ng protein were measured in 20 tape strips at both timepoints. Fold change was calculated for week 5 relative to baseline values with paired Wilcoxon testing used to assess the differences. Average ceramide levels (with standard deviation) were 344.88pmol/ng protein (84.1 pmol/ng) which rose to 459.44pmol/ng protein (86.04pmol/ng) following 5 weeks of lotion application - representing a 33% increase (p=<0.0001). 358 ceramide species measured were assigned to their respective classes with class sum values calculated at both timepoints. For 9 of the 12 classes, statistically significant increases were seen in the levels after 5 weeks of lotion application.

The results are shown in Table 3:

**Table 3**

| **Ceramide class** | **Baseline average** | **Standard deviation** | **Week 5 average** | **Standard deviation** | **Fold change** | **p-value** |
|---|---|---|---|---|---|---|
| **AdS** | 27.46 | 8.12 | 37.37 | 10.73 | 1.36 | 0.003 |
| **AH** | 47.01 | 12.7 | 64.91 | 17.14 | 1.38 | 0.001 |
| **AP** | 29.98 | 10.77 | 42.45 | 14.34 | 1.42 | 0.01 |
| **AS** | 19.29 | 6.32 | 26.73 | 7.03 | 1.39 | 0.002 |
| **EOdS** | 0.45 | 0.1 | 0.56 | 0.21 | 1.26 | 0.68 |
| **EOH** | 20.08 | 7.14 | 30.68 | 11.18 | 1.53 | <0.001 |
| **EOP** | 2.99 | 1.05 | 2.91 | 1.42 | 0.97 | 0.71 |
| **EOS** | 27.62 | 10.05 | 33.77 | 11.21 | 1.22 | 0.03 |
| **NdS** | 36 | 8.25 | 43.02 | 9.01 | 1.2 | 0.06 |
| **NH** | 56.78 | 18.42 | 76.82 | 26.23 | 1.35 | 0.004 |
| **NP** | 42.78 | 12.05 | 54.42 | 12.24 | 1.27 | 0.024 |
| **NS** | 34.46 | 10.9 | 46.28 | 14.32 | 1.34 | 0.001 |

Cholesterol (with standard deviation) was measured in the outer-most 5 tape strips at both timepoints with levels increasing from 11.54ng/µg protein (4.45ng/µg) to 12.51 ng/µg protein (4.12ng/ug), p-value = 0.081. For depths of 16-20 tapes strips values at baseline were 15.83ng/µg protein (5.25ng/µg) rising post application to 24.49ng/µg protein (8.16ng/ug), p-value <0.001.

Microbiome functional profile was examined at baseline and after 5 weeks of lotion application. Linear Discriminant Analysis was carried out using LEfSE (DOI: 10.1186/gb-2011-12-6-r60) to identify pathways that were differentially abundant at baseline and after lotion application. The following is a list of pathways that were identified as being statistically more abundant at baseline and after 5 weeks of lotion application. Statistical significance was applied to pathways with p-value < 0.01 and LDA score (log 10) >2.0.

| **Metabolic Pathways elevated at Baseline** |
|---|
| Peptidoglycan Biosynthesis IV |
| |

| **Metabolic Pathways elevated after 5 weeks of lotion application** |
|---|
| Oleate biosynthesis IV (anaerobic) |
| Palmitoleate biosynthesis i |
| Mycolate biosynthesis |
| Stearate biosynthesis ii |
| (5Z)-dodecenoate biosynthesis |
| Superpathway of fatty acid biosynthesis initiation |
| Palmitate biosynthesis |
| L-tryptophan degradation |
| NAD de novo biosynthesis |

It can be seen from the results that nine functional pathways are elevated following lotion application in comparison to baseline. Most of these pathways represent an increase in microbial production of skin-relevant fatty acids.

### Conclusion

The results demonstrate that the functional potential and interconnectivity of the microbiome are improved following 5 weeks of lotion use. This includes increases in pathways associated with biosynthesis of multiple long chain fatty acids.

Whilst some of the increase in FFA levels (C16:0 and C18:0) may be due to residual product deposition, it is likely that the increase is at least partially derived from the altered functional output of the skin microbiome (i.e., increased levels of microbially biosynthesized oleate, palmitoleate, stearate and palmitate).

Furthermore, the increase in longer chain FFAs such as, C20:0 and C22:0 is evidently not due to residual product deposition. The increase may thus be attributable to the altered functional output of the skin microbiome (i.e., increased FFA biosynthesis and elongation from shorter chain precursors).

The increases in 9/12 ceramide classes and in cholesterol also demonstrate improvements in a broad range of lipids which may be attributable to the altered functional output of the skin microbiome.

It follows that lotion use (as described above) not only effects the skin barrier directly but also can potentially alter the skin microbiome to a state which may provide additional skin care benefits, through increased levels of microbially derived lipids such as FFAs, ceramides and cholesterol.

## Claims

1. The cosmetic use of a topically applied skin care composition comprising glycerol, C₁₆₋₁₈ fatty acid and petrolatum for enhancing stratum corneum (SC) lipid biosynthesis by resident skin commensal microbes.

2. Use according to claim 1, in which the SC lipids are cholesterol, ceramides, and free fatty acids (FFAs) in which the FFAs have a linear or branched, saturated or monounsaturated aliphatic tail of at least 20 carbon atoms, such as from 20 to 32 carbon atoms.

3. Use according to claim 1 or claim 2, in which the glycerol, C16-18 fatty acid and petrolatum in the topically applied skin care composition are combined with a cosmetically acceptable vehicle, in the form of an emulsion.

4. Use according to any one of claims 1 to 3, in which the topically applied skin care composition comprises from 2 to 15% glycerol (by weight based on the total weight of the composition); from 1.5 to 4% C16-18 fatty acid (by weight based on the total weight of the composition) and from 0.25 to 5% petrolatum (by weight based on the total weight of the composition).
